(19) Europäisches Patentamt
European Patent Office
Office européen des brevets

(11) Publication number: **0 623 348 A1**

(12) # EUROPEAN PATENT APPLICATION

(21) Application number: **94107553.3**

(51) Int. Cl.$^5$: **A61K 31/70**

(22) Date of filing: **08.04.86**

This application was filed on 16 - 05 - 1994 as a divisional application to the application mentioned under INID code 60.

(30) Priority: **27.03.86 US 845627**

(43) Date of publication of application: **09.11.94 Bulletin 94/45**

(60) Publication number of the earlier application in accordance with Art.76 EPC: **0 262 125**

(84) Designated Contracting States: **AT BE CH DE FR GB IT LI LU NL SE**

(71) Applicant: **GRUBER, Harry, Edward**
**13083 Maritime Place**
**San Diego, CA 92130 (US)**

(72) Inventor: **GRUBER, Harry, Edward**
**13083 Maritime Place**
**San Diego, CA 92130 (US)**

(74) Representative: **Kolb, Helga, Dr. Dipl.-Chem. et al**
**Hoffmann, Eitle & Partner,**
**Patent-und Rechtsanwälte,**
**Arabellastrasse 4**
**D-81925 München (DE)**

(54) **The use of AICA riboside and ribavirin for the preparation of a medicament for the treatment of allergies.**

(57) A method of treating disease by increasing the excretion of cellular adenosine including diseases of the immune, nervous and vascular systems involving administering to a patient stimulators of intracellular adenosine release.

EP 0 623 348 A1

This is a Continuation-In-Part of Application Serial No. 646,785.

FIELD OF THE INVENTION

This invention relates to a method of treating various diseases that respond beneficially to increases in extracellular levels of adenosine.

BACKGROUND OF THE INVENTION

This invention was made with Government support under Grant Nos. AM-13622; RR-00827; and HL-17682 with the National Institutes of Health and the University of California. The Government has cerntain rights in this invention.

Adenosine belongs to the class of biochemicals termed purine nucleosides and is a key biochemical cell regulatory molecule, as described by Fox and Kelly in the Annual Reviews of Biochemistry, Vol. 47, p. 635, 1978, that interacts with a wide variety of cell types, and is responsible for a myriad of biological effects. For instance, adenosine is a potent vasodilator, inhibitor of immune cell function, activator of mast cell degranulation, an inhibitor of granulocyte activation, and a putative inhibitory neurotransmitter. Considering adenosine's broad spectrum of biological activity, considerable effort has been, and continues to be aimed at establishing practical therapeutic uses for the molecule, and its analogs.

Since adenosine or similar purine nucleosides are thought to act at the level of the cell plasma membrane, by binding to receptors anchored in the membrane, past work has focused on increasing the extracellular levels of the molecules. Unfortunately, adenosine or its analogs are toxic at concentrations that have to be administered to a patient to maintain an efficacious extracellular therapeutic level, thus the administration of adenosine alone is of little therapeutic use. Consequently other ways of maintaining high local extracellular levels of adenosine have been sought. The three most used are: a) interference with the uptake of adenosine with reagents that specifically block adenosine transport as described by Paterson et al., in the Annals of the New York Academy of Sciences, Vol. 255, p. 402, 1975; b) prevention of the metabolism of adenosine which, in turn, makes cellular adenosine available for extracellular transport and use outside the cell as described by Carson and Seegmiller in The Journal of Clinical Investigation, Vol. 57, p. 274, 1976; and c) the use of analogs of adenosine constructed to bind to adenosine cell plasma membrane receptors with lower dissociation constants than adenosine.

There exists a large repertoire of chemicals that inhibit the cellular uptake of adenosine. Some do so specifically, and are essentially competitive inhibitors of adenosine uptake, and others inhibit nonspecifically. Theophylline appears to be a competitive inhibitor, while dipyridamole, and a variety of other chemicals, including colchicine, phenethyalcohol and papaverine inhibit uptake nonspecifically.

Methods of increasing the extracellular levels of adenosine by altering adenosine metabolism are primarily founded on the use of chemicals that inhibit enzymatic degradation of adenosine. Most of this work has focused on identifying inhibitors of adenosine deaminase which converts adenosine to inosine. Adenosine deaminase is inhibited by coformycin, 2'-deoxyco-formycin or erythro 9-(2-hydroxy-3-nonyl) adenine hydrochloride.

A number of adenosine analogs have been generated that exhibit structural modifications to the purine ring, alterations in substituent groups attached to the purine ring, and modifications to, or alterations in the site of attachment of the carbohydrate moiety. Halogenated adenosine derivatives appear to be most promising and, as described by Wolff et al. in the Journal of Biological Chemistry, Vol. 252, p. 681, 1977, exert biological effects similar to those caused by adenosine.

Although all three techniques discussed above have advantages over the use of adenosine alone, they have several disadvantages, the major ones being that they rely on chemicals that have adverse therapeutic side effects primarily due to the fact that they must be administered in doses that are toxic, and they affect nonselectively most cell types. As described in Purine Metabolism in Man, (eds. De Baryn, Simmonds and Muller), Plenum Press, New York, 1984, most cells in the body carry receptors for adenosine. Consequently the use of techniques that increase adenosine levels generally throughout the body cause dramatic changes in normal cellular physiology in addition to controlling diseases that benefit from adenosine treatment.

Thus, it will be appreciated from the foregoing discussion that a technique that would increase extracellular levels of adenosine or adenosine analogs without complex side effects, and which would permit increased adenosine levels to be selectively targeted to cells that would benefit most from them, would be of considerable therapeutic use.

SUMMARY OF THE INVENTION

A novel method is described for alleviating disease based on increasing the extracellular concentration of adenosine, or adenosine analogs utilizing purine nucleosides, particularly the non toxic purine nucleosides, 5-amino-1-(β-D-ribofuranosyl) imidazole-4-carboxamide (AICA riboside), and 1-β-D-ribofuranosyl-1,2,4-triazole-3-carboxamide (ribavirin) and allopurinol riboside (or allopurinol base which can be converted in the body to the riboside). The latter reagents are administered to a patient and are taken up and converted to mono and sometimes triphosphate by cells. Adenosine or inosine are generated from adenosine triphosphate in the course of rapid cellular energy utilization such as during seizure activity or during decreased blood flow by a series of intracellular biochemical reactions. The production of inosine is much greater than that of adenosine (approximately 100 to 1). The compounds subsequently diffuse out of the cell and consequently are present in the immediate extracellular environment at high levels. The purine nucleosides such as AICA riboside enhance the production of adenosine. The increase in adenosine release is at least partly due to a reduction in 5'nucleotidase activity. It is apparent from the diagram below that with reduced 5'nucleotidase activity IMP is not cleaned as rapidly to inosine and more nucleotide is converted to AMP which builds up and is converted to adenosine. The $K_M$ for AMP (1mM) is approximately 10 fold higher than for IMP (1mM). Since adenosine levels are not altered significantly throughout the patient, and alterations only occuring in the areas of rapid AMP use, the method of treatment does no cause generalized adenosine release.

DIAGRAM 1

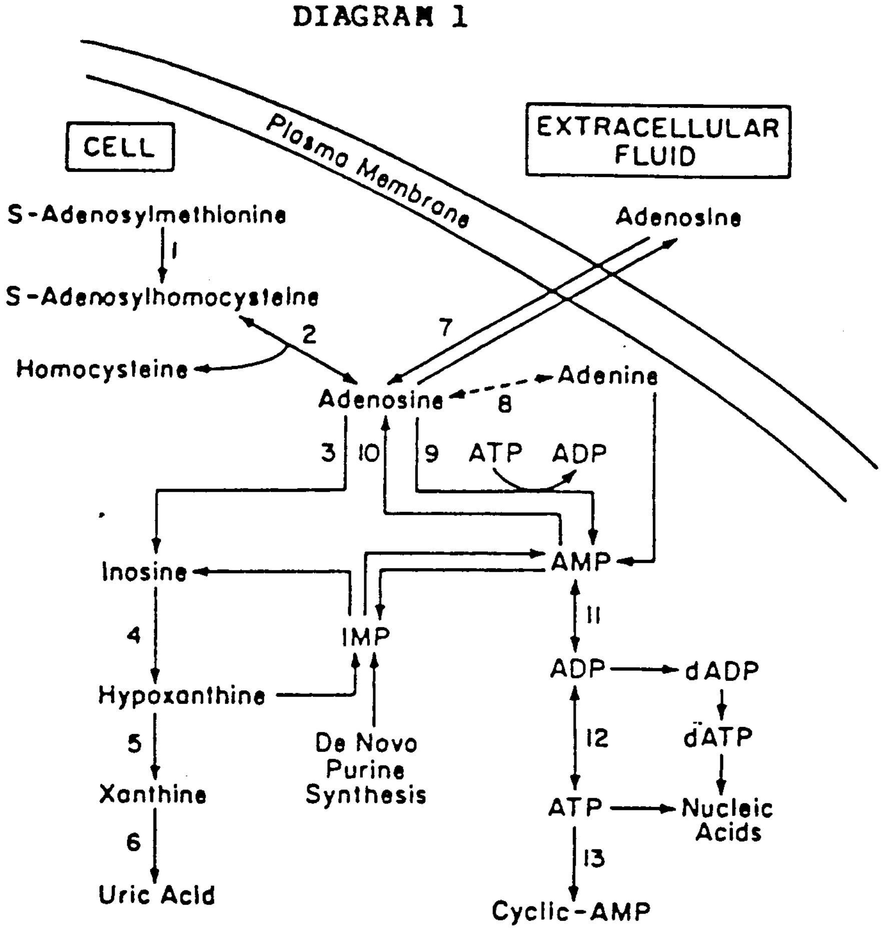


Diagram 1. Metabolism of adenosine. This diagram illustrates the pathways by which adenosine is formed and degraded with cells. Adenosine may also be transported into cells or released from cells. The metabolism of 2'-deoxyadenosine may utilize some of these pathways: 1, S-adenosylmethionine methyltransferases; 2, S-adenosylhomocysteine hydrolase; 3, adenoside deaminase; 4, purine nucleoside phosphorylase; 5 & 6, xanthine oxidase; 7, transport mechanisms; 8 adenosine phosphorylase (not established); 9, adenosine kinase; 10 5'nucleotidase and non-specific phosphatase; 11, adenylate kinase; 12, nucleoside diphosphokinase; 13, adenylate cyclase.

It is therefore apparent that the purine nucleosides, AICA riboside, ribavirin, and allopurinol afford a medically beneficial means of establishing a high local extracellular concentration of adenosine without the toxicity, or nonspecific uptake problems associated with other techniques that regulate extracellular adenosine levels.

## BRIEF DESCRIPTION OF THE DRAWINGS

Figure 1. In vitro effect of 5-amino-1-(β-D-ribofuranosyl) imidazole-4-carboxamide on adenosine excretion by lymphocytes.

Figure 2. In vivo effect of 5-amino-(1-β-D-ribofuranosyl) imidazole-4-carboxamide (AICA riboside) on regional myocardial blood flow during coronary artery occlusion in dogs. Regional myocardial blood flow was measured using radiolabelled microspheres infused into the left atrium at 5 (open) and 60 (hatched) minutes of occlusion. The means plus the standard deviations are graphed.

Figure 3. The effect of AICA riboside treatment on coronary venous adenosine concentrations. Coronary venous blood was collected into equal volumes of chilled 2N perchloric acid at various times before and after coronary artery occlusion. Supernatant from these extracts were neutralized with alamine and freon and evaluated by high performance liquid chromotography. The mean adenosine concentrations +/- standard deviations for the 5 saline treated (▭) and 6 AICA riboside treated (●) dogs are graphed.

Figure 4. β-hexosaminidase release from control and ribavirin-treated mast cells. Mouse bone marrow-derived mast dells cultured for 3-7 days in media alone (▭) or 10 M ribavirin (▨) were challenged with the calcium ionophore. The percentages of -hexosaminidase release from resting and stimulated cells are shown as means +/- SE of duplicate values from 7 experiments. * = significantly different from control cells ($p < 0.05$). Similar results were obtained with DNP-BSA antigen stimulation of anti-DNP IgE-sensitized mast cells.

Figure 5. Dose-response effects of ribavirin on mast cells -hexosaminidase release. Mast cells were cultured in media alone (controls) or 1, 10, or 20μM ribavirin for 6 days, washed, and challenged with A23187 (▭), and net β-hexosaminidase release was quantitated. Ribavirin-treated cells at all concentrations tested released significantly less β-hexosaminidase when challenged with A23187. Mediator content and spontaneous release were no different in control and ribavirin-exposed cells. Depicted are means +/- SE of duplicate determination from 3 experiments.

Figure 6. Comparison of the effects of of AICA riboside in saline (○) and saline (▭) on inosine levels.

## DETAILED DESCRIPTION OF THE INVENTION

The effect of the purine nucleosides, AICA riboside or ribavirin, on adenosine transport can be demonstrated either in vitro or in vivo where the effective concentration of either molecule is 10μM-100μM. When delivered to cells in vitro AICA riboside or ribavirin can be dissolved in aqueous solution and added directly to the culture media. To deliver these molecules to patients, it is anticipated that they will most often be administered orally since the purine nucleosides are not readily degraded by enzymes in the body, or by exposure to low pH presence in the stomach. There is no a priori reason for believing that the drug cannot also be administered intravenously, or by direct intramuscular injection, topically, or by inhalation.

Upon contact with activated energy-requiring target cells, the purine nucleosides AICA riboside or ribavirin are transported intracellularly by a plasma-membrane bound facilitated diffusion transport system where they can be phosphorylated by adenosine kinase to yield purine nucleotide monophosphate. The latter chemical can be converted to the triphosphate, an intermediate in the de novo synthesis of purine nucleosides.

In order to regulate the amount of adenosine, and hence the effectiveness of AICA riboside or ribavirin treatment, the degree of increased extracellular transport is monitored by isolating the aqueous fluid that bathes the cells, and determining the amount present in the media by chromatographic techniques as described by Matsumoto et al. in The Journal of Biological Chemistry, Vol. 254, p. 8956, 1979. In the tissue culture experiments, one millimolar deoxycoformycin was added to the aqueous fluid to prevent destruction of adenosine by adenosine deaminase. In addition, the amount of adenosine remaining in the cells is analyzed by high pressure liquid column chromatography after isolation from the cells as described by Matsumoto et al., in The Journal of Biological Chemistry, Vol. 254, p. 8956, 1979.

Since the amount of extracellular adenosine can be regulated by increasing or decreasing the concentration of the AICA riboside or ribavirin present in the cellular growth environment, if the adenosine level is too low to be medically beneficial, it can be increased by increasing the amount of AICA riboside or ribavirin administered. Similarly, if the amount of adenosine is too great, it can be reduced by reducing the

amount of the purine nucleoside.

It is anticipated that AICA riboside or ribavirin treatment will benefit patients suffering from a variety of illnesses. For example, individuals suffering from allergies, particularly asthma, hay fever, chronic urticaria, urticaria pigmentosa and eczema can be expected to benefit from purine nucleoside treatment. As discussed in B. Benacerra and A. Unanue in Textbook of Immunology (Williams & Williams, Baltimore/London, 1979), the key to suppressing allergic responses is to prevent the release of pharmacologically active substances by mast cells. Mast cells are large basophilic staining cells with extensive granules that contain substances such as histamines that are liberated by the mast cell during the allergic reaction and that are required to support the allergic response. The release of these pharmacologically active substances present in mast cells is termed "degranulation." Thus, chemicals that prevent degranulation have a beneficial effect on reducing the severity of the allergic response. As such, patients experiencing allergies can be successfully treated with AICA riboside or ribavirin as these molecules prevent mast cell degranulation. Mast cell activation causes the release of prostaglandins and leukotrienses (non-preformed mediators) such as slow reactive substance of anaphylaxis.

Additionally, patients that are suffering from autoimmune diseases may experience relief if treated with either purine nucleoside. Autoimmune diseases occur naturally in man and involve an immune reaction to an individual's own tissues. For an autoimmune response to be mounted, it is required that different immune cells interact to support the response. Thus, chemicals that interfere with the requisite cell-cell interactions can be expected to interfere with the course of the disease. An immune cell type necessary for the generation of an autoimmune response is the T-lymphocyte. Since adenosine is well-known to be toxic to T-lymphocytes, it is to be expected that administering AICA riboside or ribavirin will delete or inhibit this population of immune cells and thus be of considerable therapeutic benefit to autoimmune sufferers. Also adenosine inhibits granulocyte production of oxygen free radicals.

In addition to allergy and autoimmune diseases, it should be possible to use AICA riboside or ribavirin to treat diseases that arise from, or are aggravated by, insufficient blood flow through a particular organ. For example, angina pectoris, transient ischemic attacks, or migraine headaches can be treated by administering either purine nucleoside. This is expected since adenosine is known to be a potent vasodilator acting by reducing vascular smooth muscle contraction.

In addition to acting as a vasodilator, AICA riboside or ribavirin increase collateral blood flow by a second mechanism. Often granulocytes stick to microvasculature in the region of restricted blood flow. Both drugs cause granulocytes to dislodge, and in doing so help to reestablish normal blood flow. Thus, the uptake of AICA riboside or ribavirin by smooth muscle cells followed by subsequent release of adenosine should cause vasodilation and/or removal of granulocytes.

Another disease associated with restricted blood flow is myocardial arrhythmia. Although restricted blood flow initiates the onset of arrhythmia, the precise cause is unknown. However, it is known that lipid peroxidation by oxygen radicals is arrhythmogenic. Since the latter are secreted by granulocytes, AICA riboside or ribavirin inhibition of granulocyte activation can be expected to control arrhythmia. In addition, granulocytes are in higher concentration in areas of arteriosclerosis. Suppression of their activation might reduce the release of other mediators of arrhythmias.

The following examples, while exemplary of the present invention, should not be construed as specifically limiting the invention, and such variations which would be within the purview of one skilled in this art are to be considered to fall within the scope of this invention.

## EXAMPLE I

Regulation of mast cell degranulation by the purine nucleotide, 5-amino-1-(β-D-ribofuranosyl) imidazole-4-carboxamide (AICA riboside).

The degranulation of mast cells plays a key role in allergic diseases such as asthma. Thus, a means of preventing degranulation affords a way to control the disease.

### Mast cell isolation

To demonstrate enhanced excretion of adenosine from mast cells, the cells were first isolated from 250 gram rats by lavaging the pleural and peritoneal cavities with $Ca^{++}$ and $Mg^{++}$-free Tyrode's buffer containing 0.1g gelatin and 5mg of heparin in 100mL. After washing the cells one time by centrifugation at 100 x G in the above buffer, they were resuspended in Tyrode's buffer containing 0.1g gelatin and 1mg DNase per 100mL of buffer, and filtered through nylon bolting cloth and suspended at a concentration of $3\text{-}7X10^7$

nucleated cells/mL. Next the mast cells were purified on metrizamide gradients by suspending 2mL of the cells onto 2-mL cushions consisting of 22.5% (W/V) metrizamide in Tyrode's buffer with gelatin and DNase, and spun at 500 x G for 15 minutes at room temperature. The cell pellet was washed 2X's and resuspended at 5-10X$10^6$ nucleated cells/mL and 4-5 mL layered onto 30 mL of a 3-9% (W/V) continuous metrizamide gradient. Centrifugation at 35 x G for 12 minutes at room temperature separated the red cells from mast cells. Mast cells recovered by this technique routinely make up better than 90% of the pelleted cells.

Effect of AICA riboside on degranulation

Inhibition of degranulation by AICA riboside was demonstrated by showing that AICA riboside inhibits degranulation induced by the calcium ionophore, A23187, as reflected in the release of the acid exoglycosidase, B-hexosaminidase. A23187 at 1 microgram/millilitre, with or without AICA riboside, is added to 2-5x$10^6$ mast cells at 37ºC on Tyrode's buffer, and the amount of mast cell B-hexosaminidase released measured. In the presence of 100 micromolar AICA riboside only 17.6% of B-hexosaminidase is released, whereas 28.8% is released in its absence. Thus AICA riboside inhibits mast cell degranulation. The percent release of B-hexosaminidase, as well as the method of assaying for the enzyme was performed as described by Schwartz et al. in the Journal of Immunology, Vol. 123, October 1979, p. 1445.

EXAMPLE II

AICA riboside enhancement of adenosine release by lymphoblasts

Adenosine is a known inhibitor of certain facets of the immune response. Thus, a means of regulating adenosine concentrations enables one to beneficially treat sufferers of autoimmune deseases since they generally are a consequence of overactive immune cell reactions.

Isolation of lymphoblasts

The human splenic lymphoblast cell line, WI-L2 was used to demonstrate the effect of AICA riboside on adenosine release. The history and properties of the cell line have been described by Hershfield et al. in Science, Vol. 197, p. 1284, 1977. The cell line was maintained in RPMI 1640 cell culture media supplemented with 20% fetal calf serum and 2 nM glutamine and grown in an atmosphere of 5% carbon dioxide in air. However, because fetal calf serum contains purine metabolizing enzymes to establish the effect of AICA riboside WI-L2 cells were incubated in RPMI 1640 medium supplemented with 10% heat-inactivated, dialyzed fetal bovine serum, 2 mM glutamine, and 10 mM 4-(2-hydroxyethyl)-1β-piperazineethanesulfonic acid buffer, pH 7.4 (Calbiochem).

Effect of AICA riboside on adenosine release

Figure 1 shows that AICA riboside, over the range of 100-500 micromolar, enhances adenosine release from lymphoblasts.

About 1.4 nanamoles of adenosine/$10^6$ WI-2 cells is excreted spontaneously and this number was increased to about 2.3 nanamoles at 500 micromolar AICA riboside.

The amount of adenosine released by the cells into the supernatant, or the amount of nucleosides remaining in the cells is determined by mixing 30 microlitres of chilled 4.4N perchloric acid to the supernatants or 300 microlitres of chilled 0.4N perchloric acid to the cell pellets and centrifuging at 500 X G for 10 minutes at 4°C. Each resulting supernatant is neutralized with 660 microlitres of a solution containing 2.4 grams of tri-n-octylamine (Alamine 336) (General Mills) in 12.5 millilitres of 1,1,2-trichloro-trifluoroethone (Freon-113) solvent as described by Khym in Clinical Chemistry, Vol. 21, p. 1245, 1975. Following centrifugation at 1500 x G for 3 minutes at 4°C, the aqueous phase is removed and frozen at -20°C until assayed for adenosine, or for nucleosides. Adenosine is evaluated isocratically on a C-18 microBondapak reverse phase column equilibrated with 4 millimolar potassium phosphate, pH 3.4:acetonitrile 60%(95:5 v/v) buffer. Adenosine elutes at 8-10 minutes and its identity was confirmed by its sensitivity to adenosine deaminase and by solution with adenosine standards. The extracted samples from the cell pellet can also be analyzed for nucleosides by high pressure liquid chromatography on a Whatman Partisil-10 (SAX) column equilibrated with 10 millimolar potassium phosphate, pH 3.78, and eluted with a linear gradient to a 0.25 molar potassium phosphate, 0.5 molar KCl, pH 3.45. Continuous monitoring was performed by absorbance at 254 and 280 nm. Peaks are quantitated by comparison with high pressure liquid chromatography analysis of suitable standards.

EXAMPLE III

In Vitro effect of AICA riboside on adenosine release in neuroblastoma cells

Neuroblastoma cell lines were grown in media and under conditions described in Example II. Media was supplemented with 0.005mM - 0.5M AICA riboside and micromolar amounts of the calcium ionophore A23187. Under these conditions the cells secrete at least two-fold more adenosine than control cells.

EXAMPLE IV

In vivo effect of AICA riboside on adenosine levels and increased blood flow in dogs

Figures 2 and 3 show the results of a second series of experiments carried out to demonstrate the effects of AICA ribosides on adenosine levels in blood, and to correlate the increase in adenosine with increased blood flow. Thirteen mongrel dogs were anesthetized with phenobarbital. The anterior coronary vein was cannulated and a blood sample was collected into 2N perchloric acid. Saline or 100mM AICA riboside in saline was randomly selected for infusion into the femoral vein for 45 minutes prior to coronary artery occlusion at a rate of 1 ml/min. Coronary venous blood was collected and assayed for adenosine as described in Example 1 5 minutes prior to occlusion, and after 1, 10, 20, 30 and 50 minutes of occlusion of the left anterior descending coronary artery as well as 1 minute after reperfusion. Regional myocardial blood flow was measured with 15 $\mu$m radiolabelled spheres infused into the left atrium at 5 and 60 minutes during the ischemic period as described by Heymann et al. in Prog. C.V. Dis. 20, 55 (1977). The electrocardiogram and arterial pressure were monitored throughout the period of ischemia. Six AICA riboside treated and five saline treated dogs survived the procedure. Two of the surviving saline treated animals fibrillated. The concentration of AICA riboside in AICA riboside treated dogs immediately before occlusion was 57.4 +/- 40.2$\mu$M. The range was 4.4 to 100$\mu$M.

Figure 2 shows that adenosine levels are dramatically increased upon AICA riboside perfusion, while Figure 3 shows that regional myocardial blood flow to the ischemic myocardium was significantly greater in AICA riboside than in saline treated animals. A similar degree of difference in flow was seen in endocardium and epicardium, and there were no changes between 5 and 60 minutes of ischemia. AICA riboside did not alter flow to normal myocardium, as the non-ischemic tissue flow rates are remarkably similar between the two groups. Systemic arterial pressure and heart rate at 5 and 60 minutes showed no significant differences between the two groups of dogs. Arterial blood gas-content and system venous granulocyte counts were not significantly different between the two groups.

We conclude that AICA riboside enhances collateral coronary flow to ischemic myocardium possibly by augmenting adenosine release and thus vasodilating and/or inhibiting granulocyte capillary plugging.

EXAMPLE V

Use of 5-amino-(1-β-D-ribofuranosyl)-imidazole-4-carboxamide to treat angina.

To show the efficaciousness of AICA riboside for treating heart disease, a dog model of angina was developed. This consists of partially obstructing by placing a ring about the proximate portion of the coronary arteries feeding the dog myocardium. The ring slowly absorbs fluid and further constricts the artery over several weeks. Consequently when the dogs are exercised, they display the classical electrocardiogram and wall tension changes associated with angina. These dogs were treated with drugs known to relieve angina in humans, such as Nifedipine, and were shown to be of no help. However, when the same dogs were subsequently treated with AICA riboside, there was a dramatic, approximately 30%, increase in blood flow through the coronary arteries as measured by doppler readings. Moreover, there was also at least a two-fold increase in myocardium contractility in the ischemic region as measured by wall thickness.

EXAMPLE VI

Effect of AICA riboside treatment on arrhythmias

One consequence of myocardial ischemia is arrhythmia and the frequency of arrhythmias is related to the degree of blood flow. Therefore, the effect of AICA riboside treatment on arrhythmias was determined. Electrocardiograms recorded during ischemia were analyzed for the number of premature ventricular depolarizations (PVD) and ventricular tachycardia (VTAC) episodes. Table 1 shows that the saline treated dogs had 112.2 PVD and 18.2 episodes of VTAC during ischemia as compared to 37.8 PVD and 4.7 episodes of VTAC for the AICA treated animals (p ¼ 0.01). The one AICA riboside treated dog (#3) with frequent arrhythmias had much lower collateral blood flow rates and adenosine concentrations (but an AICA riboside blood concentration of 27.2 M) compared to the other AICA riboside treated dogs.

TABLE 1

| TREATMENT GROUP | ARRYTHIMIAS (EPISODES/H) | |
|---|---|---|
| SALINE | PVC | VTAC |
| 1 | 101 | 10 |
| 2 | 144 | 23 |
| 3 | 32 | 44 |
| 4 | 57 | 8 |
| 5 | 27 | 6 |
| AVERAGE | 118.2 | 18.2 |
| AICA RIBOSIDE | PVC | VTAC |
| 1 | 12 | 1 |
| 2 | 10 | 0 |
| 3 | 182 | 27 |
| 4 | 4 | 0 |
| 5 | 13 | 0 |
| 6 | 6 | 0 |
| AVERAGE | 37.8 | 4.7 |

Prior to ischemia none of the dogs had measurable venous adenosine (< 0.01μM) before and during AICA riboside or saline infusion. The saline treated animals had a peak adenosine level at 10 minutes after occlusion (0.22 +/-0.08μM) which fell to an undetectable level by 60 minutes. In contrast, the AICA riboside treated animals had a peak adenosine level at 1 minute of ischemia (1.79 +/- 0.35μM) which remained elevated at 60 minutes (0.18 +/- 0.15). Reperfusion resulted in no detectable adenosine washout in saline treated animals but a significant rise in the AICA treated animals.

EXAMPLE VII

Effect of Ribavirin on Mast Cell Degranulation

Bone marrow obtained from Balb/C mice femurs was cultured in a 1:1 mixture of Razin media and conditioned media, produced by co-culturing splenocytes from C57Bl/6J and C3H mice in the presence of concanavalin A as described by Razin et al. in the Proc. Natl. Acad. Sci. VSA 28i 2559-2561, 1981. After weekly passaging and at least 15 days in tissue culture, the resulting cells were 90% pure mast cells and 95% viable as assessed by Trypan blue exclusion. Cells exposed to ribavirin in culture were washed 3 times prior to use in experiments. Parallel cultures of cells grown in media alone were used as controls for pharmacologically-manipulated mast cells. Cell growth was assessed by counting cells at particular time points and comparing actual numbers of ribavirin-treated cells to numbers of cells grown in media alone.

β-hexosaminidase was chosen as a representative granule-associated, preformed mast cell mediator because it is easily quantitated and its release nearly identically parallels that of histamine. Mouse bone marrow-derived mast cells were centrifuged at 200 x g for 5 minutes, washed 3 times in Tyrode's buffer lacking divalent cataions, sensitized for 30 min. at 37°C with anti-DNP (dinitrophynyl phospate) IgE

(1$\mu$g/$10^6$ cells), and challenged with either DNP-BSA antigen (175ng/3 x $10^5$ cells) or A23187 (10$\mu$g/ml/3 x $10^5$ cells) in 400$\mu$l of complete Tyrode's buffer for 10 min. at 37 °C. Reaction mixtures were centrifuged at 200 x g for 10 min., and supernatant and pellet $\beta$-hexosaminidase concentrations were assayed by the hydrolysis of p-nitrophenyl-$\beta$-D-glucosamide as described by Schwartz et al. in J. Immunol 123, 1445 (1979). Spontaneous $\beta$-hexosaminidase release was determined in unchallenged cells. The net % of $\beta$-hexosaminidase released is defined as follows:

$$\text{Net\% } \beta\text{-hex release} = \frac{\text{super. } [\beta\text{-hex}]}{\text{super. } [\beta\text{-hex}] + \text{pellet}} - \text{spontaneous } [\beta\text{-hex}] \text{ released}$$

where [$\beta$-hex] is -hexosaminidase and super. is supernatant. When exogenous adenosine was present in reaction mixtures, it was added simultaneously with the secretagogue.

Mouse bone marrow-derived mast cells challenged with A23187 or DNP-BSA antigen released 8-15% of total cell $\beta$-hexosaminidase, a preformed, granule-associated mediator. Ribavirin (10$\mu$M) added at the time of mast cell stimulation does not affect $\beta$-hexosaminidase release. However, mast cells incubated for 3-7 days in 10$\mu$M ribavirin, washed, and challenged with A23187 exhibited a marked attenuation of $\beta$-hexosaminidase release compared to parallel cells cultured in media alone (Figure 4). Ribavirin exposure did not alter mast cell mediator content (i.e. total cell $\beta$-hexosaminidase concentration) nor cell viability, and spontaneous release of $\beta$-hexosaminidase was similar in the two cell groups. The dose-response relationship between ribavirin exposure and preformed mediator release is depicted in Figure 5. Although 1$\mu$M ribavirin for 6 days inhibits mediator release significantly, maximal inhibition is evident between 10$\mu$M and 20$\mu$M.

EXAMPLE VIII

Effect of AICA riboside treatment on inosine levels

That the increase in the levels of adenosine is due, at least in part, to a reduction in inosine levels was shown by treating dogs as described in Example IV with AICA riboside followed by analysis of venous blood for inosine levels. Figure 6 shows a more than 2 fold decrease in inosine levels over a 60 minute assay period.

EXAMPLE IX

The effect of AICA riboside treatment on coronary infarct size was determined in rats by inducing restricted blood flow by tying off the coronary artery and subsequently blousing the animals with either AICA riboside in saline, or saline or alone. Further, the animals were continuously exposed by infusion of either AICA riboside or a saline using ozmonic mini-pumps well-known to those in the art. After three weeks the rats were sacrificed and infarct size quantitated by planinarizing stained sections of fixed hearts. The results showed that in AICA riboside treated rats there is a reduction of infarct size of approximately 24% compared to saline-treated controls.

**Claims**

**1.** The use of AICA riboside and ribavirin for the preparation of a medicament for the treatment of allergies.

**2.** The use according to claim 1 for the treatment of asthma.

**3.** The use according to claim 1 for the treatment of hay fever.

**4.** The use according to claim 1 for the treatment of urticaria.

**5.** The use according to claim 1 for the treatment of urticaria pigmentosa.

**6.** The use according to claim 1 for the treatment of eczema.

**7.** The use according to any of the preceding claims, wherein AICA riboside and ribavirin is administered orally, intramuscularly, topically or by inhalation.

**8.** The use according to any of the preceding claims, wherein AICA riboside and ribavirin is administered intravenously.

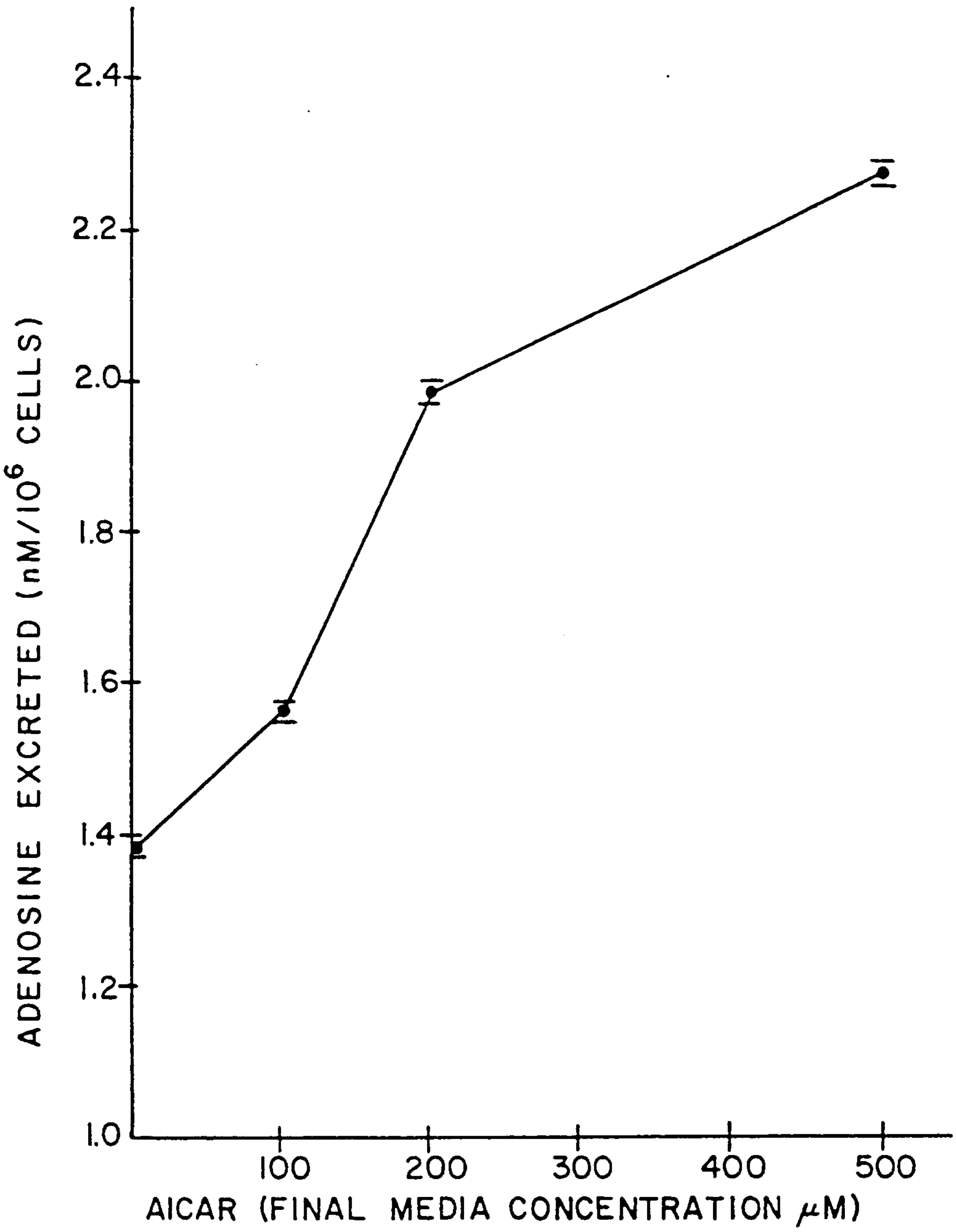
2.4
2.2
2.0
1.8
1.6
1.4
1.2
1.0
ADENOSINE EXCRETED (nM/10⁶ CELLS)
100
200
300
400
500
AICAR (FINAL MEDIA CONCENTRATION µM)

FIG. 1

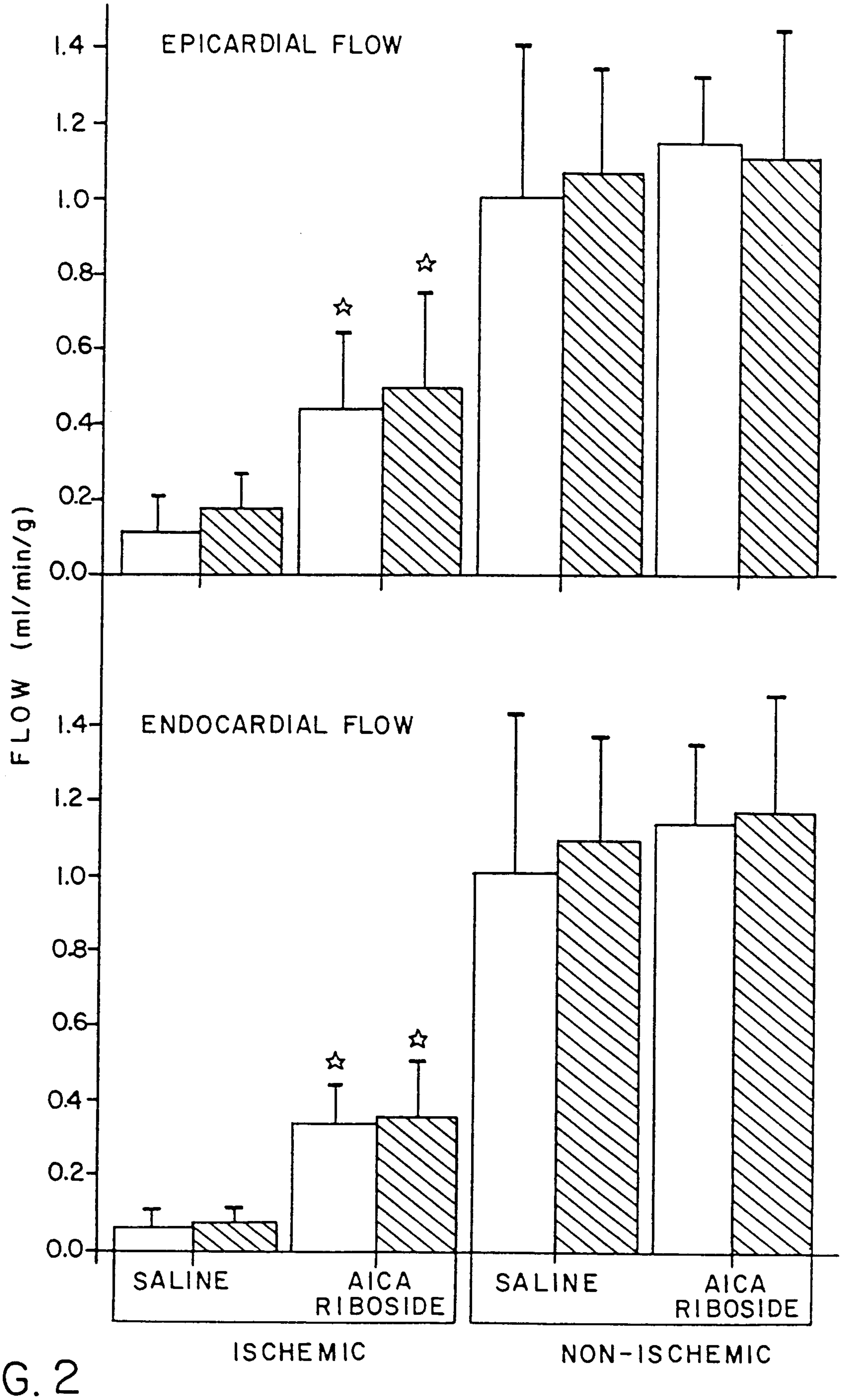
EPICARDIAL FLOW
ENDOCARDIAL FLOW
FLOW (ml/min/g)
0.0
0.2
0.4
0.6
0.8
1.0
1.2
1.4
SALINE
AICA RIBOSIDE
SALINE
AICA RIBOSIDE
ISCHEMIC
NON-ISCHEMIC

FIG. 2

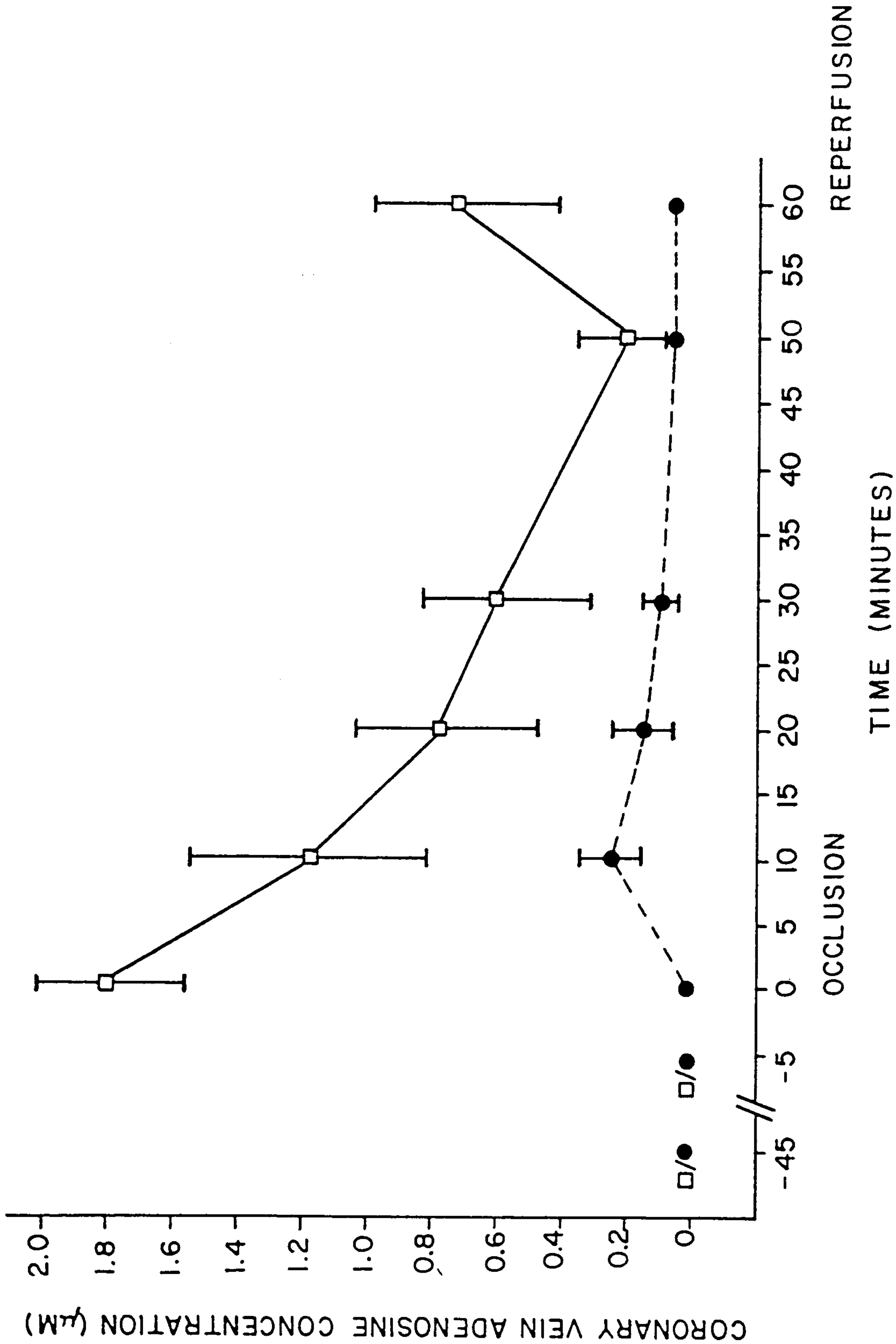
CORONARY VEIN ADENOSINE CONCENTRATION (μM)
2.0
1.8
1.6
1.4
1.2
1.0
0.8
0.6
0.4
0.2
0
-45
-5
0
5
10
15
20
25
30
35
40
45
50
55
60
OCCLUSION
REPERFUSION
TIME (MINUTES)

FIG. 3

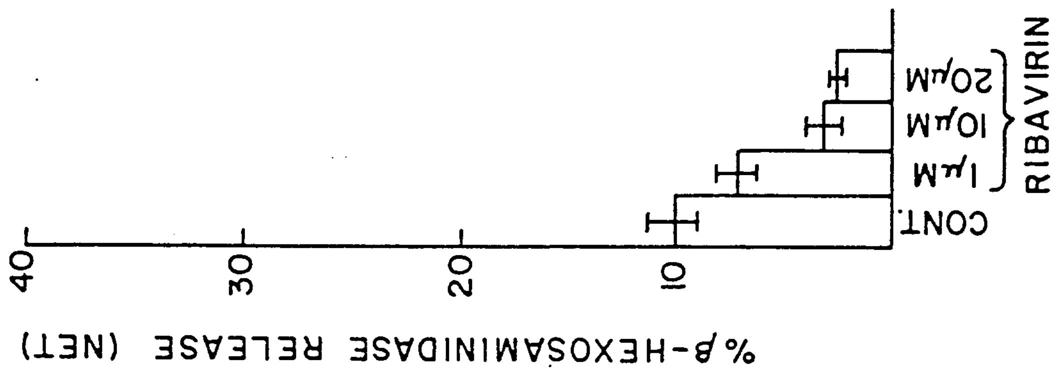
% β-HEXOSAMINIDASE RELEASE (NET)
40
30
20
10
CONT.
1μM
10μM
20μM
RIBAVIRIN

FIG. 5

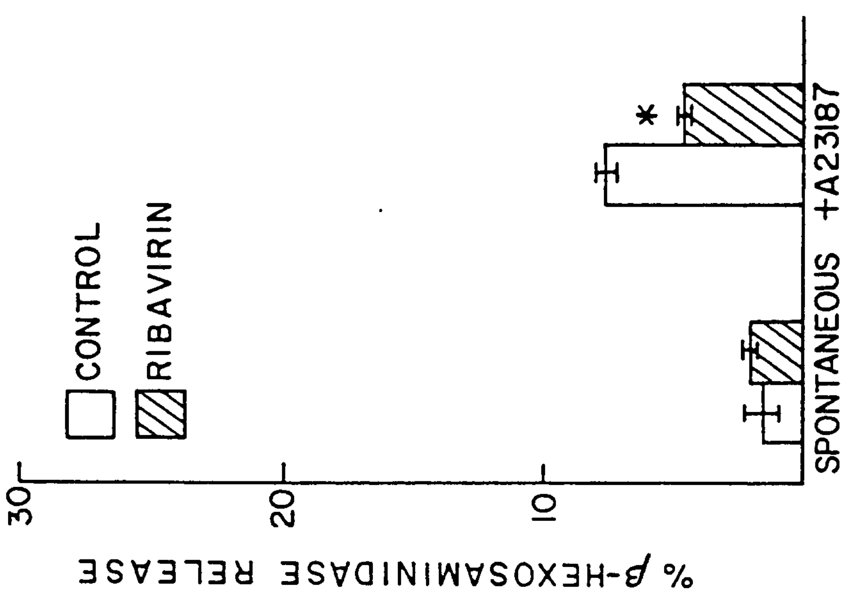
% β-HEXOSAMINIDASE RELEASE
30
20
10
CONTROL
RIBAVIRIN
*
SPONTANEOUS
+A23187

FIG. 4

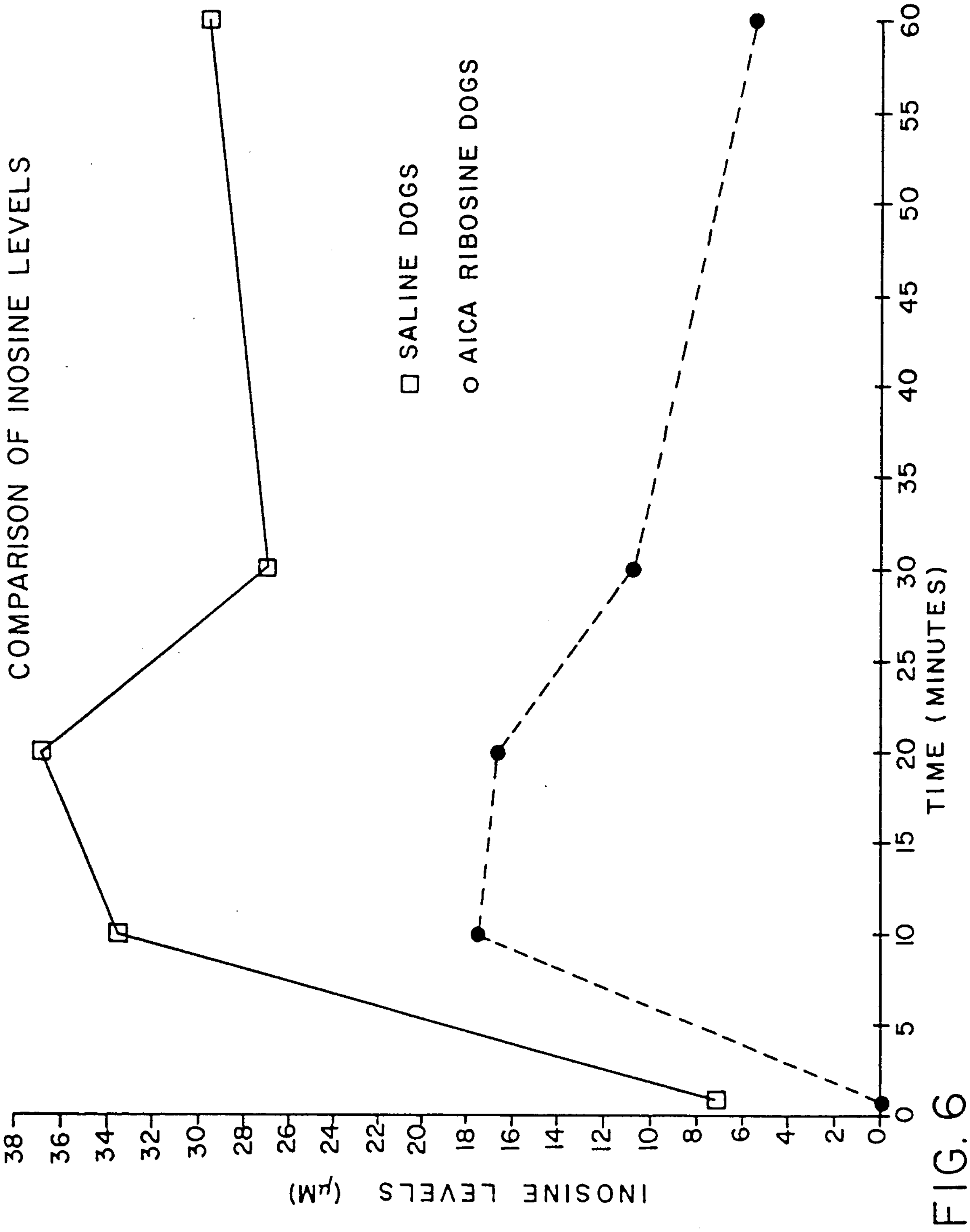
COMPARISON OF INOSINE LEVELS
SALINE DOGS
AICA RIBOSINE DOGS
INOSINE LEVELS (µM)
38
36
34
32
30
28
26
24
22
20
18
16
14
12
10
8
6
4
2
0
0
5
10
15
20
25
30
35
40
45
50
55
60
TIME (MINUTES)

FIG. 6

European Patent Office

# EUROPEAN SEARCH REPORT

Application Number
EP 94 10 7553

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| X | ADV.EXP. MED. BIOL.<br>vol. 195, no. PT.B , 1986<br>pages 15 - 18<br>D. MARQUARDT ET AL. 'Inhibition of mast cell mediator release by 5-amino4-imidazolecarboxamide riboside'<br>* page 15, last paragraph *<br>--- | 1-6 | A61K31/70 |
| Y | AGENTS AND ACTIONS<br>vol. 10, no. 1/2 , 1980<br>pages 145 - 147<br>B. FREDHOLM ET AL. 'Histamine and Immunology'<br>* page 147, left column *<br>--- | 1-8 | |
| Y | LIFE SCIENCES<br>vol. 18 , 1976<br>pages 153 - 162<br>J. TATESON ET AL. 'Inhibition of adenosine-3',5'-cyclic monophosphate phosphodiesterase by potential antiallergic compounds'<br>* the whole document *<br>--- | 1-8 | TECHNICAL FIELDS SEARCHED (Int.Cl.4)<br>A61K |
| Y | US-A-4 575 498 (HOLMES)<br>* the whole document *<br>--- | 1-8 | |
| A | US-A-4 211 771 (WITKOWSKI)<br>* the whole document *<br>--- | 1-8 | |
| T | J. PHARMACOL. EXP. THER.<br>vol. 240, no. 1 , 1987<br>pages 145 - 149<br>D. MARQUARDT ET AL. 'Ribavirin inhibits mast cell mediator release'<br>* the whole document *<br>---<br>-/-- | 1-6 | |

The present search report has been drawn up for all claims

| Place of search | Date of completion of the search | Examiner |
|---|---|---|
| MUNICH | 29 July 1994 | Foerster, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 94 10 7553

## DOCUMENTS CONSIDERED TO BE RELEVANT

| Category | Citation of document with indication, where appropriate, of relevant passages | Relevant to claim | CLASSIFICATION OF THE APPLICATION (Int.Cl.4) |
|---|---|---|---|
| T | BIOCHEM. PHARMACOL. vol. 35, no. 24 , December 1986 pages 4415 - 4421 D. MARQUARDT ET AL. 'Inhibition of mast cell mediator release by 5-amino-4-imidazolecarboxamide riboside' * the whole document * ----- | 1-6 | |
| | | | TECHNICAL FIELDS SEARCHED (Int.Cl.4) |

The present search report has been drawn up for all claims

| Place of search | Date of completion of the search | Examiner |
|---|---|---|
| MUNICH | 29 July 1994 | Foerster, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)